# EUROPEAN PATENT APPLICATION

(11) **EP 4 424 297 A1**
(43) Date of publication of application: **04.09.2024**
(21) Application number: 22886851.9
(22) Date of filing: 20.10.2022
(51) Int. Cl.: A61K 8/27, A61Q 7/00, A61K 8/86, A61K 8/73

(54) **COSMETIC FOR HAIR GROWTH, RAW MATERIAL FOR COSMETIC, COSMETIC, AND METHODS FOR PRODUCING THESE**

(30) Priority: 26.10.2021 JP 2021174344
(71) Applicant: Teika Pharmaceutical Co., Ltd., Toyama-shi, Toyama 930-0982 (JP)
(72) Inventor: YAMAKAWA, Tetsumi, Toyama-shi, Toyama 930-0982 (JP); MATSUMOTO, Saori, Toyama-shi, Toyama 930-0982 (JP); MATSUI, Masafumi, Toyama-shi, Toyama 930-0982 (JP); KUBO, Tetsufumi, Toyama-shi, Toyama 930-0982 (JP); SHIGA, Takuro, Toyama-shi, Toyama 930-0982 (JP)
(74) Representative: Mewburn Ellis LLP
(86) International application number: PCT/JP2022/039084
(87) International publication number: WO 2023/074523

(57) **Abstract**

An object of the present invention is to provide a novel application of simonkolleite. The cosmetic for hair growth of the present invention comprises simonkolleite crystals as an active ingredient.

## Description

### Technical Field

The present invention relates to a cosmetic raw material and a cosmetic and particularly relates to a cosmetic raw material and a cosmetic for use in hair growth or scalp care.

### Background Art

Simonkolleite is zinc chloride hydroxide hydrate composed of zinc, a hydroxy group, and chlorine and is a compound represented by the chemical formula Zn₅(OH)₈Cl₂·(H₂O)ₙ. Simonkolleite is a white crystalline powder insoluble in water and an organic solvents and is also called basic zinc chloride or zinc chloride hydroxide. Simonkolleite, which is also a natural mineral, was disclosed as a novel mineral in 1985 and named after Werner Simon and Kurt Kolle who collected mineral samples of simonkolleite. Simonkolleite has been utilized as a main component that constitutes tetrabasic zinc chloride (TBZC) serving as a feed additive for an animal and reported as a nutritional supplement preferable for animals.

In recent years, research and development have been underway on novel applications or industrial production methods of this simonkolleite. For example, Patent Literature 1 reports a technique using simonkolleite as a therapeutic agent for wound skin or damaged skin. Patent Literature 2 reports a method for producing simonkolleite capable of being used as an active pharmaceutical ingredient.

On the other hand, the efficacy of compounds containing zinc has been reported in the fields of hair care and scalp care. For example, Patent Literature 3 reports that zinc salts such as zinc gluconate, zinc chloride, and zinc sulfate have trypsin inhibitory activity and are therefore useful in preventing or reducing hair loss. Patent Literature 4 describes a composition for hair growth containing *Scutellaria baicalensis* root extract which is a pilatory and/or hair growth component(s), zinc gluconate, and bis-ethoxyglycol cyclohexane dicarboxylate, and states that among them, zinc gluconate has an effect of imparting firmness and elasticity to hair. Non Patent Literature 1 reports results of studying a zinc complex compound pyrithione zinc for its effect on hair density and hair thickness.

### Citation List

### Patent Literature

Patent Literature 1: Japanese Patent No. 6185215
Patent Literature 2: International Publication No. WO 2018/105738
Patent Literature 3: Japanese Translation of PCT International Application Publication No. 2016-540806
Patent Literature 4: Japanese Patent No. 6797673

### Non Patent Literature

Non Patent Literature 1: R. S. Berger et al., "The effects of minoxidil, 1% pyrithione zinc and a combination of both on hair density: a randomized controlled trial", British Journal of Dermatology, Vol. 149, No. 2, 2003, p. 354-362

### Summary of Invention

### Technical Problem

Zinc salts such as zinc gluconate, zinc chloride, and zinc sulfate reported in Patent Literatures 3 and 4 are substances that have high solubility in water and are dissolved in water to quickly generate a zinc ion. This zinc ion prevents or reduces hair loss and exerts an effect of imparting firmness and elasticity to hair. Non Patent Literature 1, which may support this, reports that, although pyrithione zinc having very low solubility in water was studied for its effect on hair density and hair thickness, the pyrithione zinc was observed to have only less than half the effect of a positive control minoxidil. Simonkolleite elutes a very small amount of a zinc ion into water, as in pyrithione zinc. Simonkolleite has therefore not been expected to have efficacy as reported in Patent Literatures 3 and 4. Hence, the use of simonkolleite for hair care and scalp care has not yet been studied, and its effectiveness has been totally unclear.

On the other hand, simonkolleite crystals that are used in Patent Literature 1 or produced by a production method introduced in Patent Literature 2 as mentioned above, or other generally distributed simonkolleite crystals have been found to have a primary particle size as large as approximately 10 µm (D₅₀: median size) and also have aggregability. The inventors of the present application have attempted to prepare a cosmetic by dispersing such simonkolleite crystals in a dispersion medium such as water or ethanol and blending the dispersion with other cosmetic raw materials generally used, and consequently faced a problem of the occurrence of irreversible or difficult-to-redisperse aggregation and precipitation.

Simonkolleite crystals are a white powder having a large particle size. Therefore, the simonkolleite crystals, when applied to the skin not only directly but as a dispersion in a dispersion medium such as water or ethanol, raise perceivable "gritty feel" and cause white "residues on the skin". Such physical properties are likely to be unpopular to users and are big drawbacks, particularly, for use as a cosmetic raw material. Particularly, in the case of blending simonkolleite into a cosmetic for scalp care, white "residues on the skin" caused by simonkolleite crystals extremely differs in color from hair when the cosmetic is applied to the scalp. Therefore, such residues are conspicuous in the hair and might be confused with "scurf" and thus take on a very unfavorable posture in terms of beauty care.

Thus, the present invention has been made in light of the points mentioned above. An object of the present invention is to provide a novel application of simonkolleite.

Another object of the present invention is to provide a cosmetic raw material and a cosmetic containing simonkolleite crystals that are easily redispersed without aggregating even when dispersed in a dispersion medium such as water or ethanol, and are easy to handle.

A further alternative object of the present invention is to provide a cosmetic raw material and cosmetics containing simonkolleite crystals that reduce "gritty feel" and white "residues on the skin" upon use and offer smooth feeling of use and favorable appearance in terms of beauty care even after use.

### Solution to Problem

The inventors of the present application have studied various applications as to simonkolleite crystals and consequently found that the simonkolleite crystals have an effect of promoting hair elongation surpassing minoxidil. On the basis of this finding, the present invention has been completed. In order to solve the problem described above, the cosmetic for hair growth of the present invention comprises simonkolleite crystals as an active ingredient. The simonkolleite crystals promote hair elongation in follicle tissues. Therefore, healthy growth of hair is maintained, and healthy hair can be obtained.

An amount of the simonkolleite crystals blended in the cosmetic for hair growth of the present invention is preferably 0.001% by weight to 10% by weight. A concentration of the simonkolleite crystals that is superior in hair growth effect is thereby selected.

The simonkolleite crystals in the cosmetic for hair growth of the present invention are also preferably nanoparticles having a 90% cumulative particle size (D₉₀) of 800 nm or smaller in a volume-based particle size distribution obtained by a laser diffraction scattering method. The simonkolleite crystals are nanoparticles having D₉₀ of 800 nm or smaller, whereby gritty feel caused by insoluble simonkolleite crystals serving as an active ingredient when the cosmetic is applied to the scalp is dissolved, in addition, while white residues on the skin no longer occur. Therefore, the cosmetic for hair growth that offers smooth feeling of use and favorable appearance in terms of beauty care even after use is obtained.

The cosmetic for hair growth of the present invention further comprises a dispersion medium and a dispersant, wherein the dispersant is also preferably at least one compound selected from the group consisting of polyethylene glycol, polypropylene glycol, an ethylene oxide-propylene oxide copolymer, hydroxypropyl cellulose, hydroxypropyl methylcellulose, and hydroxyethyl cellulose. When the dosage form of the cosmetic for hair growth is a slurry (suspension) of the simonkolleite crystals dispersed in the dispersion medium, the simonkolleite crystals are thereby hardly to aggregate, possess enhanced stability on storage, and become easily redispersible. Therefore, the resulting cosmetic for hair growth is also easy to handle.

The cosmetic raw material or the cosmetic of the present invention comprises nanoparticles of simonkolleite crystals. A novel cosmetic raw material and cosmetic that are cosmetically useful are thereby obtained. The simonkolleite crystals are used as nanoparticles, whereby gritty feel caused by the simonkolleite crystals is reduced, in addition the white residues on the skin are hardly to occur. Therefore, the resulting cosmetic can offer smooth feeling of use and favorable appearance in terms of beauty care even after use. In the present description, the nanoparticles refer to particulate substances having a 90% cumulative particle size (D₉₀) of 1 nm or larger and smaller than 1000 nm from a small particle side in a volume-based particle size distribution obtained by a laser diffraction scattering method.

The nanoparticles of simonkolleite crystals in the cosmetic raw material or the cosmetic of the present invention also preferably have a 90% cumulative particle size (D₉₀) of 800 nm or smaller in a volume-based particle size distribution obtained by a laser diffraction scattering method. The gritty feel and the white residues on the skin caused by the simonkolleite crystals thereby no longer occur when the cosmetic is applied to the skin. Therefore, the resulting cosmetic offers more smooth feeling of use and more favorable appearance in terms of beauty care even after use.

The nanoparticles of simonkolleite crystals in the cosmetic raw material or the cosmetic of the present invention also preferably have a 50% cumulative particle size (D₅₀) of 250 nm or smaller in a volume-based particle size distribution obtained by a laser diffraction scattering method. The gritty feel and the white residues on the skin caused by the simonkolleite crystals thereby no longer occur when the cosmetic is applied to the skin. Therefore, the resulting cosmetic offers much more smooth feeling of use and much more favorable appearance in terms of beauty care even after use.

The cosmetic raw material or the cosmetic of the present invention further comprises water, a water-miscible organic solvent, or a combination thereof as a dispersion medium. The resulting cosmetic raw material or cosmetic is thereby in the form of a slurry (suspension).

The cosmetic raw material or the cosmetic of the present invention also preferably further comprises a dispersant. The simonkolleite crystals contained in the slurry are thereby hardly to aggregate, possess enhanced stability on storage, and are easily redispersed. Therefore, the resulting cosmetic raw material or cosmetic is also easy to handle.

The dispersant mentioned above is also preferably at least one compound selected from the group consisting of polyethylene glycol, polypropylene glycol, an ethylene oxide-propylene oxide copolymer, hydroxypropyl cellulose, hydroxypropyl methylcellulose, and hydroxyethyl cellulose. A compound suitable as the dispersant is thereby selected.

An amount of the simonkolleite crystals blended in the cosmetic raw material or the cosmetic of the present invention is also preferably 0.001% by weight to 30% by weight. A concentration of the simonkolleite crystals suitable for the cosmetic raw material or the cosmetic is thereby selected.

The cosmetic raw material or the cosmetic of the present invention is also preferably for hair growth or for scalp care use. The simonkolleite crystals have an effect of promoting hair elongation in follicle tissues and maintaining healthy growth of hair. Therefore, an application suitable for the cosmetic raw material or the cosmetic is selected.

The method for producing a cosmetic raw material or a cosmetic according to the present invention comprises: a mixing process of mixing simonkolleite crystals with a dispersion medium to obtain a suspension; a stirring process of stirring this suspension for 1 hour or longer; and a downsizing treatment process of introducing the suspension after the stirring process to a pulverizer, and downsizing the simonkolleite crystals contained in the suspension into a nano-level size. Through the stirring process of stirring the suspension of the simonkolleite crystals and the dispersion medium for 1 hour or longer so that both the components are warmed up, the clogging of a pipeline such as a flow path or a nozzle in the pulverizer is hardly to occur, and the downsizing treatment of the simonkolleite crystals is made easier. Therefore, the resulting cosmetic raw material or cosmetic contains the simonkolleite crystals downsized into a nano-level size.

In the method for producing a cosmetic raw material or a cosmetic according to the present invention, it is also preferred that in the mixing process or the stirring process, the suspension should be further mixed with a dispersant. In the mixing process or the stirring process, the dispersant is added, and the simonkolleite crystals and the dispersant are warmed up into the dispersion medium, whereby the downsizing treatment of the simonkolleite crystals with the pulverizer is facilitated while the resulting cosmetic raw material or cosmetic contains the simonkolleite crystals that are hardly to aggregate and also possess improved dispersibility.

The method for producing a cosmetic raw material or a cosmetic according to the present invention comprises: a mixing process of mixing simonkolleite crystals with a dispersion medium having a water-miscible organic solvent to obtain a suspension; a downsizing treatment process of introducing the suspension to a pulverizer, and downsizing the simonkolleite crystals contained in the suspension into a nano-level size; and a process of evaporating the water-miscible organic solvent contained in the suspension after the downsizing treatment. By use of the water-miscible organic solvent as the dispersion medium, the clogging of a pipeline such as a flow path or a nozzle in the pulverizer is hardly to occur. Therefore, the downsizing treatment can be performed by introducing the suspension of the dispersion medium and the simonkolleite crystals to the pulverizer without performing the stirring process for warming up both the components. In this respect, the water-miscible organic solvent is evaporated from the obtained suspension so that the water-miscible organic solvent can be replaced with another solvent, specifically with water. Therefore, the resulting cosmetic raw material or cosmetic is composed of an aqueous suspension (slurry) of the downsized simonkolleite crystals.

In the method for producing a cosmetic raw material or a cosmetic according to the present invention, it is also preferred that in the mixing process, the suspension should be obtained by further mixing with a dispersant. By the addition of the dispersant, the resulting cosmetic raw material or cosmetic contains the simonkolleite crystals that are hardly to aggregate and also possess improved dispersibility.

### Advantageous Effects of Invention

The present invention can provide a cosmetic for hair growth having the following excellent effects.
(1) Simonkolleite crystals promote hair elongation. Therefore, healthy growth of hair is maintained, and healthy hair is obtained.
(2) The cosmetic has a long duration of the effects and can therefore be less frequently used.
(3) The simonkolleite crystals serving as an active ingredient are nanoparticles, whereby gritty feel and white residues on the skin caused by the simonkolleite crystals no longer occur. Therefore, the cosmetic offers smooth feeling of use and favorable appearance in terms of beauty care even after use.
(4) A dispersant is blended, whereby the simonkolleite crystals are hardly to aggregate, possess enhanced stability on storage, and become easily redispersible. Therefore, the cosmetic is also easy to handle.

The present invention can also provide a cosmetic raw material or a cosmetic having the following excellent effects and a method for producing the same.
(1) The simonkolleite crystals are nanoparticles. Therefore, gritty feel caused by the simonkolleite crystals is reduced while white residues on the skin are also hardly to occur. The resulting cosmetic can offer smooth feeling of use and favorable appearance in terms of beauty care even after use.
(2) A dispersant is blended, whereby the simonkolleite crystals in a slurry are hardly to aggregate, possess enhanced stability on storage, and are easily redispersed. Therefore, the resulting cosmetic raw material or cosmetic can also be easy to handle.
(3) The simonkolleite crystals have an effect of promoting hair elongation and maintaining healthy growth of hair and as such, can be suitably used in a cosmetic raw material or a cosmetic for hair growth or for the scalp.
(4) A cosmetic raw material or a cosmetic in the form of a slurry containing the simonkolleite crystals downsized into a nano-level size can be easily obtained.

### Brief Description of Drawings

[Figure 1] Figure 1 is a graph showing hair elongation by an organ culture method of rat whisker follicle in Example 4.
[Figure 2] Figure 2 is a graph showing time-dependent change in particle size distribution of simonkolleite crystals in Example 6.
[Figure 3] Figure 3 is a graph showing hair elongation by an organ culture method of rat whisker follicle in Example 9.

### Description of Embodiments

Hereinafter, the cosmetic for hair growth, the cosmetic raw material, and the cosmetic, and the method for producing the same according to the present invention will be described.

The simonkolleite crystals for use in the cosmetic for hair growth, the cosmetic raw material, or the cosmetic of the present invention refer to a white crystalline powder of zinc chloride hydroxide hydrate represented by Zn₅(OH)₈Cl₂·(H₂O)ₙ. Simonkolleite crystals industrially produced by, for example, a method described in Patent Literature 2 mentioned above are generally distributed. As one example, a simonkolleite product of JFE Mineral & Alloy Co., Ltd. is suitably used. Simonkolleite obtained by any of other production methods or simonkolleite derived from a natural mineral may be used.

The simonkolleite crystals to be blended into the cosmetic for hair growth of the present invention are preferably nanoparticles in order to reduce gritty feel caused by the simonkolleite crystals upon application to the scalp and white residues on the skin after application. In the present description, the nanoparticles refer to particulate substances having a 90% cumulative particle size (D₉₀) of 1 nm or larger and smaller than 1000 nm from a small particle side in a volume-based particle size distribution obtained by a laser diffraction scattering method. In the present invention, the simonkolleite crystals are preferably nanoparticles having a 90% cumulative particle size (D₉₀) of 800 nm or smaller, more preferably nanoparticles having a 50% cumulative particle size (D₅₀) of 250 nm or smaller, further preferably nanoparticles having a 50% cumulative particle size (D₅₀) of 150 nm or smaller, particularly preferably nanoparticles having a 90% cumulative particle size (D₉₀) of 550 nm or smaller and a 50% cumulative particle size (D₅₀) of 150 nm or smaller, from the viewpoint of more reducing gritty feel and white residues on the skin caused by the simonkolleite crystals.

According to the present invention, the hair growth application, i.e., the cosmetic for hair growth or the cosmetic raw material for hair growth, refers to a cosmetic or a cosmetic raw material having an effect of promoting hair elongation in follicle tissues, and means a cosmetic or a cosmetic raw material that can promote hair elongation in follicle tissues as compared with a control without the application or addition of the cosmetic or the cosmetic raw material for hair growth of the present invention. The level of promotion of hair elongation in follicle tissues may be measured by a method known in the art, for example, an organ culture method of rat whisker follicle.

The amount of the simonkolleite crystals contained in the cosmetic for hair growth of the present invention may be appropriately set depending on a targeted hair growth effect, a formulation, a dosage form, a use method, the number of uses, etc., and is preferably 0.001% by weight to 10% by weight, more preferably 0.005% by weight to 5% by weight, further preferably 0.01% by weight to 3% by weight, from the viewpoint of an effective activity thereof.

On the other hand, the simonkolleite crystals to be blended into the cosmetic raw material or the cosmetic of the present invention are nanoparticles. Gritty feel caused by the simonkolleite crystals upon application to the skin and white residues on the skin after application are thereby reduced. As for the particle size of the nanoparticles, specifically, the simonkolleite crystals are preferably nanoparticles having a 90% cumulative particle size (D₉₀) of 800 nm or smaller, more preferably nanoparticles having a 50% cumulative particle size (D₅₀) of 250 nm or smaller, further preferably nanoparticles having a 50% cumulative particle size (D₅₀) of 150 nm or smaller, particularly preferably nanoparticles having a 90% cumulative particle size (D₉₀) of 550 nm or smaller and a 50% cumulative particle size (D₅₀) of 150 nm or smaller, from the viewpoint of more reduing gritty feel and white residues on the skin caused by the simonkolleite crystals.

The cosmetic for hair growth, the cosmetic raw material, or the cosmetic of the present invention is preferably a suspension (slurry) of the simonkolleite crystals dispersed in a dispersion medium, from the viewpoint of ease of handling. Water, a water-miscible organic solvent, or a combination thereof is used as the dispersion medium. Examples of the water-miscible organic solvent include organic solvents miscible with water, for example, alcohols such as ethanol and propanol, ethers such as tetrahydrofuran, esters such as methyl acetate, and ketones such as acetone. Ethanol is suitably used from the viewpoint of safety, etc.

For the cosmetic for hair growth, the cosmetic raw material, or the cosmetic prepared as a suspension (slurry), it is preferred to further blend a dispersant in order to prevent the formation of aggregation and precipitation that block the simonkolleite crystals from being easily redispersed in the suspension. The dispersant is an ethylene oxide polymer, an ethylene oxide-propylene oxide copolymer, a cellulose derivative, or a combination thereof. Specifically, polyethylene glycol, polypropylene glycol, an ethylene oxide-propylene oxide copolymer, hydroxypropyl cellulose, hydroxypropyl methylcellulose, or hydroxyethyl cellulose is suitably used, though the dispersant is not particularly limited thereto. Among them, hydroxypropyl cellulose, hydroxypropyl methylcellulose, polyethylene glycol 6000, or a combination thereof is more suitably used from the viewpoint of more improving the dispersibility of the simonkolleite crystals in the suspension. The amount of the dispersant blended in the suspension is preferably 0.05% by weight to 10% by weight, more preferably 0.1% by weight to 5% by weight, from the viewpoint of improving dispersibility.

The concentration of the simonkolleite crystals to be contained in the cosmetic raw material of the present invention is preferably 1% by weight to 30% by weight, more preferably 5% by weight to 20% by weight, from the viewpoint of improvement in distributiveness of the cosmetic raw material and ease of handling at the time of production. On the other hand, the concentration of the simonkolleite crystals to be contained in the cosmetic of the present invention may be appropriately set depending on a targeted application (function) and effect, a formulation, a dosage form, a use method, etc. Specifically, one example thereof is preferably 0.001% by weight to 10% by weight, more preferably 0.005% by weight to 5% by weight, further preferably 0.01% by weight to 3% by weight, though the concentration is not particularly limited thereto.

As for the application of the cosmetic raw material or the cosmetic of the present invention, the cosmetic raw material or the cosmetic can be used for hair growth or for scalp care because the simonkolleite crystals have a hair growth effect, as mentioned above. Furthermore, the cosmetic raw material or the cosmetic may be used for the purpose of exerting other functions possessed by the simonkolleite crystals.

Next, the method for producing a cosmetic for hair growth, a cosmetic raw material, or a cosmetic according to the present invention will be described. The simonkolleite crystals to be blended thereinto are nanoparticles, whereas generally distributed simonkolleite crystals have an average particle size (D₅₀) as large as approximately 10 µm. Hence, it is necessary to downsize the simonkolleite crystals into a nano-level size. For downsizing treatment, wet milling is performed because of being excellent in downsizing into a nano-level size. Hereinafter, the details will be described.

### (Mixing process)

First, simonkolleite crystals are mixed with a dispersion medium to obtain a suspension. Water, a water-miscible organic solvent, or a combination thereof is used as the dispersion medium, as mentioned above. In this mixing process, it is also preferred to add the dispersant mentioned above so that the dispersant is contained in the suspension. A slurry that possesses more improved dispersibility can be obtained by allowing the dispersant to be contained in the suspension containing the simonkolleite crystals, introducing the suspension to a pulverizer, and performing downsizing treatment of the simonkolleite crystals.

### (Stirring process)

Next, the suspension obtained by mixing the simonkolleite crystals with the dispersion medium is stirred. By this stirring process, the simonkolleite crystals in the suspension are warmed up to the dispersion medium. Therefore, the clogging of a pipeline such as a flow path or a nozzle in a pulverizer is hardly to occur during downsizing treatment, and the downsizing treatment of the simonkolleite crystals is facilitated. The stirring unit is not particularly limited as long as the whole suspension can be stirred. For example, a propeller stirrer or a magnetic stirrer can be used. The stirring time is preferably 1 hour or longer, more preferably 2 hours or longer, in order to warm up the dispersion medium and the simonkolleite crystals. In this stirring process, it is also preferred to add the dispersant mentioned above so that the dispersant is contained in the suspension. In the case of selecting a water-miscible organic solvent such as ethanol as the dispersion medium, the downsizing treatment process may be performed without the stirring process.

### (Downsizing treatment process)

In the downsizing treatment process, the suspension is introduced to a pulverizer, and the simonkolleite crystals in the suspension are downsized into a nano-level size. A wet pulverizer is used as the pulverizer, and, for example, a high-pressure homogenizer as a wet pulverizer or a bead mill as a wet pulverizer is used. The downsizing treatment is preferably performed such that the simonkolleite crystals become nanoparticles having a 90% cumulative particle size (D₉₀) of 800 nm or smaller, more preferably performed such that the simonkolleite crystals become nanoparticles having a 50% cumulative particle size (D₅₀) of 250 nm or smaller, further preferably performed such that the simonkolleite crystals become nanoparticles having a 50% cumulative particle size (D₅₀) of 150 nm or smaller, particularly preferably performed such that the simonkolleite crystals become nanoparticles having a 90% cumulative particle size (D₉₀) of 550 nm or smaller and a 50% cumulative particle size (D₅₀) of 150 nm or smaller. For the downsizing treatment, the high-pressure homogenizer-type apparatus requires downsizing treatment conditions such as the chamber used, a pressure, a nozzle diameter, the number of passes, or a treatment time, and the bead mill-type apparatus requires downsizing treatment conditions such as a material of beads used, a bead diameter, or a treatment time. These treatment conditions may be appropriately set on the basis of the specification of the apparatus used and the desired downsizing level. In this downsizing treatment process, preliminary milling treatment of milling the simonkolleite crystals in the suspension beforehand may be performed, followed by downsizing treatment of further downsizing the crystals. The preliminary milling treatment can employ the same apparatus as the pulverizer mentioned above. The simonkolleite crystals in the suspension are roughly milled by decreasing a treatment time, the number of passes, a pressure, or the like or increasing a nozzle diameter of the chamber used, the bead diameter used, or the like, as compared with the conditions related to the downsizing treatment as the present treatment. Simonkolleite crystals having a small 50% cumulative particle size (D₅₀) and a uniform nanoparticle size can thereby be stably obtained.

### (Solvent evaporation process)

In the case of selecting a water-miscible organic solvent such as ethanol as the dispersion medium, the water-miscible organic solvent contained in the water-miscible organic solvent slurry after the downsizing treatment is evaporated so that the dispersion medium can be replaced with another dispersion medium, specifically with water. Specifically, as one example, water is added to an ethanol slurry after the downsizing treatment, and a reduced-pressure environment is created where steam distillation is then performed. Ethanol can thereby be evaporated to obtain an aqueous slurry in which the simonkolleite crystals subjected to the downsizing treatment are dispersed.

The slurry obtained by the downsizing treatment process or the evaporation process mentioned above can be used directly in the cosmetic for hair growth, the cosmetic raw material, or the cosmetic. In addition, a concentrate obtained by concentration treatment, a fine powder obtained by drying treatment, or the like may be used in the cosmetic for hair growth, the cosmetic raw material, or the cosmetic.

If the simonkolleite crystals in the blended components are not prepared as fine nanoparticles in the cosmetic for hair growth, the cosmetic for hair growth may be obtained by adding the simonkolleite crystals and optionally the dispersant to the dispersion medium, and dispersing the crystals.

The cosmetic raw material of the present invention can be used as a raw material for hair growth and for scalp care in order to promote hair elongation in follicle tissues, maintain healthy growth of hair, and obtain healthy hair, and as a raw material for an topical preparation for the skin. The cosmetic of the present invention can be used for hair growth and for scalp care in order to promote hair elongation in follicle tissues, maintain healthy growth of hair, and obtain healthy hair, and as an topical preparation for the skin.

The amount of the cosmetic for hair growth of the present invention used varies depending on a targeted hair elongation effect, a use method, age, the state of hair, etc., and cannot therefore be generalized. The amount is preferably 0.01 to 1000 µg/cm²·day, more preferably 0.1 to 500 µg/cm²·day, in terms of the simonkolleite crystals per skin unit area (1 cm²).

The cosmetic for hair growth, the cosmetic raw material, or the cosmetic of the present invention can be applied to dosage forms of various topical preparations as dosage forms usually applicable to the scalp or the skin by conventional methods. Examples thereof include slurries, liquid preparations such as low viscous liquids, gels, emulsions, pastes, creams, foams, ointments, and powders. The cosmetic for hair growth, the cosmetic raw material, or the cosmetic according to the present invention can be applied to any of cosmetic products, quasi-drugs, and medicinal product. Specific examples of such a product include, but are not particularly limited to, hair tonics, lotions for the scalps, shampoos, conditioners, treatments, styling products, soaps, lotions, cosmetic creams, cosmetic emulsions, beauty care liquids, cosmetic packs, cosmetic cleansers, bath agents, and makeup cosmetics.

Various components that are usually used in cosmetics or topical preparations can be blended into the cosmetic for hair growth, the cosmetic raw material, or the cosmetic of the present invention without impairing the effective activity of the present invention. Examples thereof include vasodilators, blood circulation promoting agents, surfactants, antihistaminic agents, anti-inflammatory agents, thickeners, vitamins, amino acids, moisturizing agents, preservatives, antimicrobial agents, fragrances, and dyes. A hair growth component or a hair regrowth component other than the simonkolleite crystals may be blended thereinto. Examples of such other hair growth or hair regrowth components include minoxidil, adenosine, and *Swertia japonica* extract.

### Examples

Hereinafter, the present invention will be described in more detail with reference to Examples and Comparative Examples. A method for measuring the particle size of simonkolleite crystals in each sample prepared in the following Examples and Comparative Examples and methods for evaluating each sample are as described below.

### (1) Particle size

The particle size distribution of each sample obtained in Examples and Comparative Examples was measured using a laser diffraction scattering-type particle size distribution measurement apparatus (model number: Partica LA-960, a product of HORIBA, Ltd.). In the measurement, the refractive index parameter of simonkolleite crystals was set to 1.700-0.000i, and ion-exchange water or anhydrous ethanol containing dispersed simonkolleite crystals was used as a dispersion medium in each sample. A 90% cumulative particle size (D₉₀) and a 50% cumulative particle size (D₅₀) in a volume-based particle size distribution were determined from the obtained particle size distribution.

### (2) Gritty Feel

Each sample obtained in Examples and Comparative Examples was diluted using the same dispersion medium (ion-exchange water or anhydrous ethanol) as that blended into the sample such that the concentration of simonkolleite crystals was 5 wt% to prepare each sample for evaluation. A 50 µL aliquot of this sample for evaluation was collected, added dropwise to the inner forearm, gently spread with a diameter of 4 cm by a finger, and evaluated for the perception of gritty feel according to the following evaluation criteria.
Excellent (indicated by double circle): Gritty feel was not perceived.
Good (indicated by circle): Gritty feel was slightly perceived.
Fair (indicated by triangle): Gritty feel was lightly perceived.
Poor (indicated by x-mark): Gritty feel was strongly perceived.

### (3) Residues on skin

The sample liquid for evaluation in the range spread on the inner forearm in the evaluation of "(2) Gritty feel" mentioned above was dried on the skin and then evaluated for the presence or absence of white residues on the skin according to the following evaluation criteria.
Excellent (indicated by double circle): No white residues on the skin were observed.
Good (indicated by circle): Few white residues on the skin were observed.
Fair (indicated by triangle): Partial white residues on the skin were slightly observed.
Poor (indicated by x-mark): White residues on the skin were evidently observed.

### (4) Dispersibility

An 8 mL aliquot of each sample for evaluation prepared by dilution such that the concentration of simonkolleite crystals was 5 wt% was collected and placed in a 10 mL vial (Mighty Vial No. 3, a product of Maruemu Corp.). The vial was shaken to disperse the simonkolleite crystals in the sample for evaluation. Then, the vial was left standing at room temperature for 18 hours. The dispersibility of the simonkolleite crystals was evaluated 18 hours later according to the following evaluation criteria. Excellent (indicated by double circle): The simonkolleite crystals maintained their dispersed state or were dispersed by merely shaking the vial lightly several times.
Good (indicated by circle): The simonkolleite crystals were dispersed by shaking the vial.
Fair (indicated by triangle): The simonkolleite crystals were dispersed by vigorously shaking the vial.
Poor (indicated by x-mark): The simonkolleite crystals remained at the bottom of the vial even by vigorously shaking the vial, and some portions were difficult to disperse.

Table 1 shows the specification of the blended components used in the following Examples and Comparative Examples.

**[Table 1]**

| Abbreviated name for component | Component name | Model number and manufacturer of product | Remarks |
|---|---|---|---|
| - | Simonkolleite | JFE Mineral & Alloy Co., Ltd. | Lot Nos: TOL81, S1C231, T0l71-A5, S1J201 |
| - | Ethanol | Anhydrous ethanol of Japanese Pharmacopoeia | |
| EEA | 2-Ethoxyethyl acetate | Tokyo Chemical Industry Co., Ltd. | |
| PGMA | Propylene glycol 1-methoxy-2-acetate | Tokyo Chemical Industry Co., Ltd. | |
| PEG400 | Polyethylene glycol 400 | PEG-400 Sanyo Chemical Industries, Ltd. | Molecular weight: 400 (number-average molecular weight) |
| PEG1000 | Polyethylene glycol 1000 | PEG-1000 Sanyo Chemical Industries, Ltd. | Molecular weight: 1000 (number-average molecular weight) |
| PEG6000a | Polyethylene glycol 6000 | PEG-6000P Sanyo Chemical Industries, Ltd. | Molecular weight: 8600 (number-average molecular weight) |
| PEG6000b | Polyethylene glycol 6000 | Kishida Chemical Co., Ltd. | Molecular weight: 7400 to 9000 |
| HPC | Hydroxypropyl cellulose | HPC-L Nippon Soda Co., Ltd. | Molecular weight: 140000 (GPC) |
| HPMC | Hydroxypropyl methylcellulose | TC-5 (R) Shin-Etsu Chemical Co., Ltd. | Molecular weight: 32800 |
| Phenoxyethanol | 2-Phenoxyethanol | Cica Grade 1 Kanto Chemical Co., Inc. | Conforming item of the Japanese standards of quasi-drug ingredients |

### [Example 1]

### 1. Preparation of aqueous slurry of simonkolleite

Simonkolleite (JFE Mineral & Alloy Co., Ltd., lot No: S1C231) was sifted through a 32-mesh sieve to obtain 5 g of a sieved product of simonkolleite. Ion-exchange water was added thereto as a dispersion medium to adjust the amount to 100 mL, followed by ultrasonic irradiation for 2 minutes to obtain 100 mL of an aqueous slurry (aqueous suspension) of simonkolleite. The particle size of the simonkolleite crystals in this slurry was a 90% cumulative particle size (D₉₀) of 9458 nm and a 50% cumulative particle size (D₅₀) of 5600 nm.

### [Example 2]

### 2. Preparation of aqueous slurry of simonkolleite nanocrystals - (1)

To 840 g of ion-exchange water, 160 g of simonkolleite (JFE Mineral & Alloy Co., Ltd., lot No: TOL81) was added and mixed, and the mixture was stirred at a stirring rate of 300 to 500 rpm for 4 hours and 30 minutes using a propeller stirrer. The aqueous slurry (aqueous suspension) of simonkolleite crystals thus stirred was introduced to a high-pressure homogenizer as a wet pulverizer (Star Burst Labo, a product of Sugino Machine Ltd.) and subjected to downsizing treatment under conditions of chamber used: ball collision chamber, chamber nozzle diameter: 0.14 mm, pressure: 245 MPa, and the number of passes: 30 passes. As a result, an aqueous slurry containing the simonkolleite crystals downsized into a nano-level size was obtained. The particle size of the simonkolleite crystals in this slurry was a 90% cumulative particle size (D₉₀) of 401 nm and a 50% cumulative particle size (D₅₀) of 133 nm.

### [Example 3]

### 3. Preparation of aqueous slurry of simonkolleite nanocrystals - (2)

To 62.5 g of the aqueous slurry obtained in Example 2, ion-exchange water was added to adjust the amount to 200 mL. The mixture was well shaken to prepare a diluted slurry having a simonkolleite crystal concentration of 5 w/v% in the slurry.

### [Comparative Example 1]

### 4. Preparation of supernatant of aqueous slurry of simonkolleite nanocrystals

Simonkolleite is insoluble in water and reportedly elutes a very small amount of a zinc ion into water. Accordingly, in order to examine the influence of the zinc ion eluted into an aqueous slurry of simonkolleite nanocrystals, a supernatant of the aqueous slurry was prepared. Specifically, the aqueous slurry having a simonkolleite crystal concentration of 5 w/v% obtained in Example 3 was treated by centrifugation (model number: AX-521, a product of Tomy Seiko Co., Ltd.) at 1830 × g for 30 minutes. A supernatant portion was recovered and filtered through a membrane filter having a pore size of 0.22 µm (Millex(R)-GV filter, a product of Merck KGaA) to obtain a clear colorless filtrate as an aqueous slurry supernatant.

Table 2 below shows the blends, production conditions, particle sizes, and various evaluations of the samples prepared in Examples 1 to 3 and Comparative Example 1.

**[Table 2]**

| | | Example 1 | Example 2 | Example 3 | Comparative Example 1 |
|---|---|---|---|---|---|
| Simonkolleite concentration | | 5wt/v% | 16wt/wt% | 5wt/v% | Supernatant of Example 3 |
| Simonkolleite LOT | | S1C231 | TOL81 | TOL81 | |
| Dispersion medium | | Water | Water | Water | |
| Dispersant | | Absent | Absent | Absent | |
| Dispersant concentration | | | | | |
| Stirring process | | Absent | Present (4.5hrs) | Present (4.5hrs) | |
| Downsizing treatment conditions | Treatment unit | Downsizing treatment not performed | High-pressure homogenizer | High-pressure homogenizer | |
| | The number of passes | | 30 | 30 | |
| | Pressure (MPa) | | 245 | 245 | (filtrate obtained by centrifuging slurry obtained in Example 3, followed by filtration through filter having pore size of 0.22 µm) |
| | Chamber | | Ball collision type | Ball collision type | |
| | Nozzle diameter (mm) | | 0.14 | 0.14 | |
| Particle size | D₅₀ (nm) | 5600 | 133 | 133 | |
| | D₉₀ (nm) | 9458 | 401 | 401 | |
| Evaluation | Gritty feel | Poor | Excellent | Excellent | |
| | Residues on skin | Poor | Excellent | Excellent | |
| | Dispersibility | Fair | Fair | Fair | |
| Remarks | | - | - | Prepared by diluting sample of Example 2 | |

### [Example 4]

### 5. Study on hair growth effect of simonkolleite crystals - (1)

The organ culture of rat whisker follicle was performed using the samples prepared in Examples 1 and 3 and Comparative Example 1 to examine a hair growth promoting effect of simonkolleite. The test was conducted as follows: a 7-week-old male SD rat having a body weight of 210 to 230 g (Japan SLC, Inc.) was anesthetized, and the skin of a whisker area was aseptically excised. Follicles having two hairs per follicle (growing follicles) were selected from among the first and second lines of whisker in the excised skin, and isolated under a stereomicroscope. The isolated follicles were washed with a culture solution of DMEM medium (Thermo Fisher Scientific Co., Ltd.) supplemented with penicillin G (100 IU/mL), streptomycin (100 µg/mL), and amphotericin B (0.25 µg/mL). Next, the samples prepared in Examples 1 and 3 and Comparative Example 1 and a positive control minoxidil were each added to DMEM medium containing 10% FBS to prepare culture solutions. As shown in Table 3 below, a culture solution for each test group (Tests 4a to 4e) was prepared by adding the samples and minoxidil shown in Table 3 below to DMEM medium containing 10% FBS (negative control) so as to attain their respective predetermined concentrations.

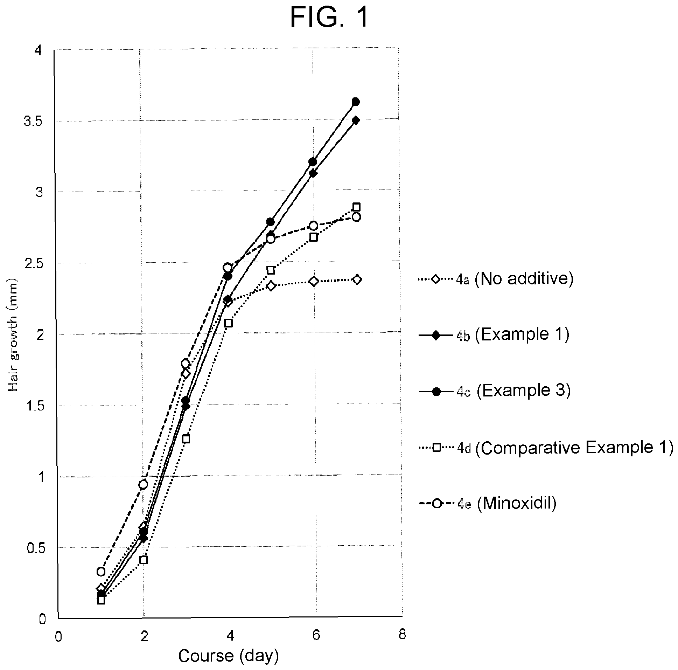

A cell culture insert (Millicell(R), a product of Merck KGaA) was put on a 6-well cell culture plate, and the membrane was wetted from the undersurface with 2 mL of the culture solution for each test group (Tests 4a to 4e). Twenty follicles per test group were randomly selected from the follicles dipped in PBS, transferred onto the membrane using tweezers, placed in a CO₂ incubator (Napco 7100), and cultured under conditions of 37°C and 5% CO₂. The culture was performed for 7 days, and hair elongation was observed and recorded as change in whole length from the starting point of melanocyte to the hair cut portion and length of the exposed hair portion from skin surface to the hair cut portion under a stereomicroscope. The results are shown in Figure 1.

**[Table 3]**

| Test No. | Sample added to culture solution | Sample concentration in culture solution |
|---|---|---|
| 4a | Negative control: no additive | - |
| 4b | Example 1: aqueous suspension of simonkolleite | Simonkolleite concentration: 5 µg/mL (0.01% of Example 1) |
| 4c | Example 3: aqueous suspension of simonkolleite nanocrystals | Simonkolleite concentration: 5 µg/mL (0.01% of Example 3) |
| 4d | Comparative Example 1: aqueous suspension supernatant of simonkolleite nanocrystals | 0.5% of Comparative Example 1 |
| 4e | Positive control: minoxidil | Minoxidil concentration: 50µM |

As shown in Figure 1, all the test groups exhibited similar hair elongation from the start of the test to the fourth day, whereas only the simonkolleite test group (4b) of Example 1 and the simonkolleite nanocrystal test group (4c) of Example 3 exhibited marked hair elongation since 4 days after the start of the test. This elongation effect stood out as compared not only with the negative control test group (4a) but with the positive control minoxidil test group (4e). No such standout effect was observed in the simonkolleite supernatant test group (4d) of Comparative Example 1. These results indicate that the marked hair elongation effect exhibited by the simonkolleite test group (4b) of Example 1 and the simonkolleite nanocrystal test group (4c) of Example 3 is not merely brought about by the effect of the zinc ion eluted into an aqueous phase held in equilibrium with simonkolleite, and rather, is an effect unique to the simonkolleite crystals which are a powder insoluble in water.

### [Example 5]

### 6. Study on dispersibility of simonkolleite nanocrystal slurry - (1)

The not downsized simonkolleite (Example 1) has a particle size distribution as large as D₅₀ of 5.6 µm and D₉₀ of 9.5 µm. Hence, gritty feel caused by crystals is perceived when a cosmetic containing such simonkolleite is applied to the skin. In addition, after application, white residues are found at the application site. These are not favorable in terms of beauty care. Since the simonkolleite nanocrystal slurries obtained in Examples 2 and 3 are nanoparticles, gritty feel and white residues on the skin are dissolved. However, aggregation and precipitation of simonkolleite crystals occur. This requires great care because, upon use, a container must be vigorously shaken until the simonkolleite crystals are redispersed. Accordingly, in order to obtain a simonkolleite nanocrystal slurry that possessed improved dispersibility from not downsized simonkolleite, various samples were prepared under blends and production conditions shown in Tables 4 and 5 below, and studied for dispersants.

### (Test 5-1)

In 103 g of anhydrous ethanol of the Japanese Pharmacopoeia, 2 g of hydroxypropyl cellulose was dissolved as a dispersant. To this solution, 20 g of simonkolleite (JFE Mineral & Alloy Co., Ltd., lot No: TOL81) was added to obtain a suspension. This suspension was introduced to a high-pressure homogenizer as a wet pulverizer (Star Burst Mini, a product of Sugino Machine Ltd.) and subjected to downsizing treatment under conditions of chamber used: ball collision chamber, chamber nozzle diameter: 0.1 mm, pressure: 200 MPa, and the number of passes: 10 passes to obtain an ethanol slurry. The particle size measurement of simonkolleite crystals and various sample evaluations were performed as to this slurry sample. The results are shown in Table 4.

### (Test 5-2)

An ethanol slurry was obtained in the same manner as in Test 5-1 except that: in Test 5-1, the amount of anhydrous ethanol blended and the amount of hydroxypropyl cellulose blended were changed to 101 g and 4 g, respectively; and the number of passes in the downsizing treatment was changed to 20 passes. The particle size measurement of simonkolleite crystals and various sample evaluations were performed as to this slurry sample. The results are shown in Table 4.

### (Test 5-3)

In 1212 g of anhydrous ethanol of the Japanese Pharmacopoeia, 48 g of hydroxypropyl cellulose was dissolved as a dispersant. To this solution, 240 g of simonkolleite (JFE Mineral & Alloy Co., Ltd., lot No: S1C231, a 30-mesh sieved product) was added to obtain a suspension. This suspension was introduced to a high-pressure homogenizer as a wet pulverizer (Star Burst Labo, a product of Sugino Machine Ltd.) and subjected to downsizing treatment under conditions of chamber used: ball collision chamber, chamber nozzle diameter: 0.14 mm, pressure: 200 MPa, and the number of passes: 30 passes to obtain an ethanol slurry. The particle size measurement of simonkolleite crystals and various sample evaluations were performed as to this slurry sample. The results are shown in Table 4.

### (Test 5-4)

In 80.8 g of anhydrous ethanol of the Japanese Pharmacopoeia, 3.2 g of hydroxypropyl cellulose was dissolved as a dispersant. To this solution, 16 g of simonkolleite (JFE Mineral & Alloy Co., Ltd., lot No: TOL81) was added to obtain a suspension. This suspension was introduced to a high-pressure homogenizer as a wet pulverizer (Star Burst Mini, a product of Sugino Machine Ltd.) and subjected to downsizing treatment under conditions of chamber used: slit chamber, chamber nozzle diameter: corresponding to 0.16 mm, pressure: 175 MPa, and the number of passes: 20 passes to obtain an ethanol slurry. The particle size measurement of simonkolleite crystals and various sample evaluations were performed as to this slurry sample. The results are shown in Table 4.

### (Test 5-5)

An ethanol slurry was obtained in the same manner as in Test 5-4 except that the number of passes in the downsizing treatment of Test 5-4 was changed to 30 passes. The particle size measurement of simonkolleite crystals and various sample evaluations were performed as to this slurry sample. The results are shown in Table 4.

### (Test 5-6)

In 82.4 g of anhydrous ethanol of the Japanese Pharmacopoeia, 1.6 g of hydroxypropyl methylcellulose was dissolved as a dispersant. To this solution, 16 g of simonkolleite (JFE Mineral & Alloy Co., Ltd., lot No: TOL81) was added to obtain a suspension. This suspension was introduced to a high-pressure homogenizer as a wet pulverizer (Star Burst Mini, a product of Sugino Machine Ltd.) and subjected to downsizing treatment under conditions of chamber used: slit chamber, chamber nozzle diameter: corresponding to 0.16 mm, pressure: 175 MPa, and the number of passes: 30 passes to obtain an ethanol slurry. The particle size measurement of simonkolleite crystals and various sample evaluations were performed as to this slurry sample. The results are shown in Table 4.

### (Test 5-7)

An ethanol slurry was obtained in the same manner as in Test 5-6 except that the dispersant in Test 5-6 was changed to polyethylene glycol 6000 (PEG-6000P, a product of Sanyo Chemical Industries, Ltd.). The particle size measurement of simonkolleite crystals and various sample evaluations were performed as to this slurry sample. The results are shown in Table 5.

### (Test 5-8)

Anhydrous ethanol of the Japanese Pharmacopoeia was mixed with 0.8% by weight of polyethylene glycol 6000 (PEG6000, a product of Kishida Chemical Co., Ltd.) and 16% by weight of simonkolleite (JFE Mineral & Alloy Co., Ltd., lot No: T0I71-A5) to obtain a suspension. This suspension was introduced to a bead mill as a wet pulverizer (Labo Star Mini LMZ015, a product of Ashizawa Finetech Ltd.) and subjected to downsizing treatment by performing milling for 150 minutes using PSZ beads of 0.2 mm in diameter, then further adding polyethylene glycol 6000 so as to attain a final concentration of 1.6% by weight, changing the PSZ beads of 0.2 mm in diameter to PSZ beads of 0.05 mm in diameter, and further performing milling for 120 minutes. The particle size measurement of simonkolleite crystals and various sample evaluations were performed as to the obtained ethanol slurry sample. The results are shown in Table 5.

### (Test 5-9)

An ethanol slurry was obtained in the same manner as in Test 5-1 except that: in Test 5-1, the amount of anhydrous ethanol blended was changed to 101 g; the dispersant was changed to polyethylene glycol 400; and the amount of the dispersant blended was changed to 4 g. The particle size measurement of simonkolleite crystals and various sample evaluations were performed as to this slurry sample. The results are shown in Table 5.

### (Test 5-10)

An ethanol slurry was obtained in the same manner as in Test 5-1 except that in Test 5-1, the dispersant was changed to polyethylene glycol 1000. The particle size measurement of simonkolleite crystals and various sample evaluations were performed as to this slurry sample. The results are shown in Table 5.

### (Test 5-11)

An ethanol slurry was obtained in the same manner as in Test 5-1 except that: in Test 5-1, the amount of anhydrous ethanol blended was changed to 152 g; the dispersant was changed to a mixture of polyethylene glycol 1000 and propylene glycol 1-methoxy-2-acetate (weight ratio: PEG1000:PGMA = 8:2); and the amount of the dispersant blended was changed to 8 g; and the amount of simonkolleite blended was changed to 40 g. The particle size measurement of simonkolleite crystals and various sample evaluations were performed as to this slurry sample. The results are shown in Table 5.

### (Test 5-12)

An ethanol slurry was obtained in the same manner as in Test 5-1 except that: in Test 5-1, the amount of anhydrous ethanol blended was changed to 78 g; the dispersant was changed to propylene glycol 1-methoxy-2-acetate; and the amount of the dispersant blended was changed to 2 g. The particle size measurement of simonkolleite crystals and various sample evaluations were performed as to this slurry sample. The results are shown in Table 5.

### (Test 5-13)

An ethanol slurry was obtained in the same manner as in Test 5-1 except that: in Test 5-1, the amount of anhydrous ethanol blended was changed to 78 g; the dispersant was changed to ethoxyethyl acetate; and the amount of the dispersant blended was changed to 2 g. The particle size measurement of simonkolleite crystals and various sample evaluations were performed as to this slurry sample. The results are shown in Table 5.

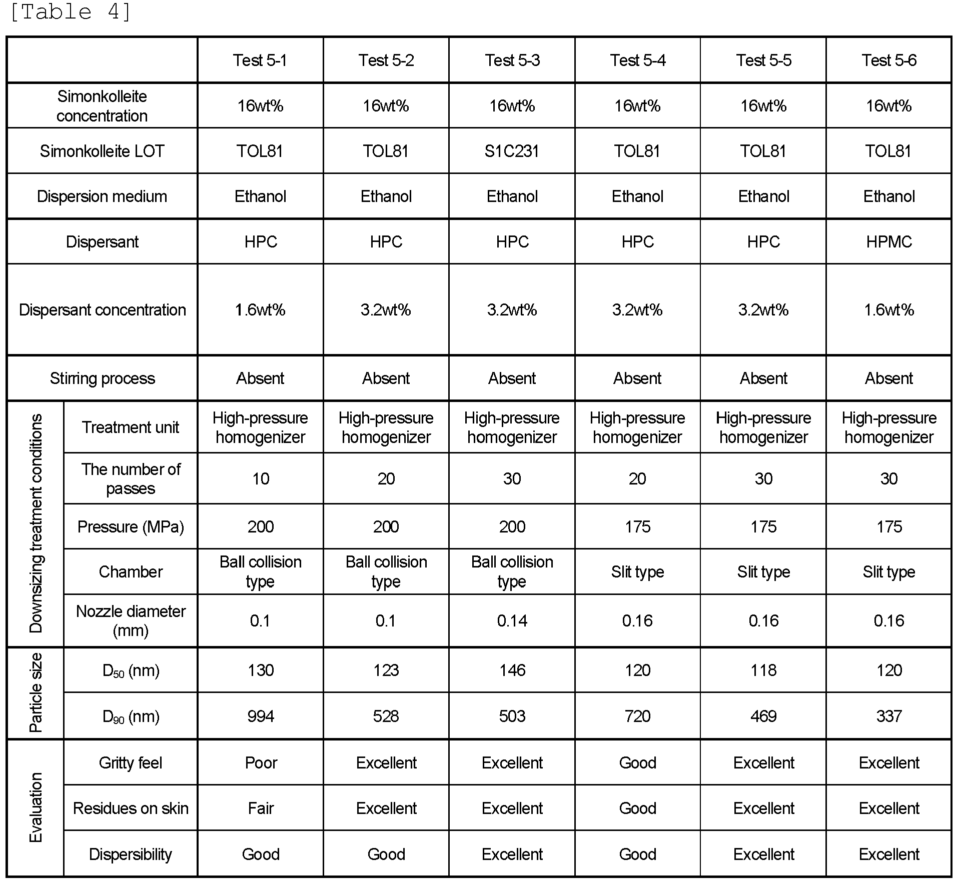

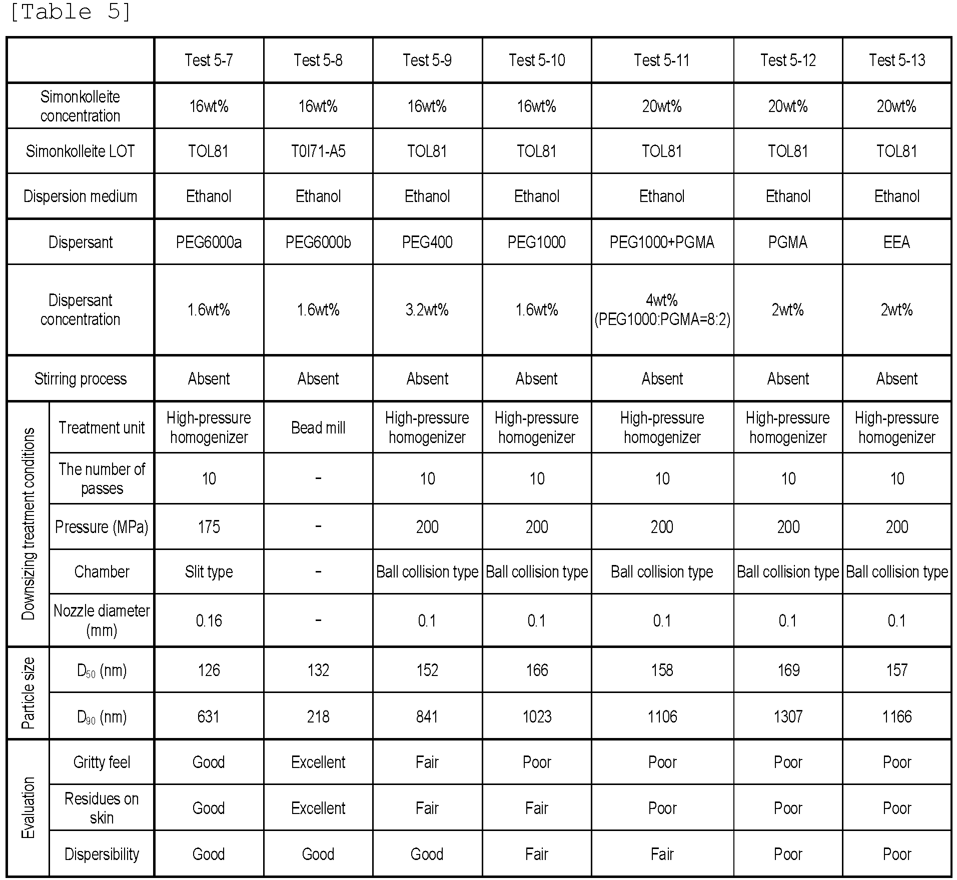

From the results of Table 4, use of cellulose derivatives hydroxypropyl cellulose and hydroxypropyl methylcellulose as the dispersant was found to improve dispersibility and produce a simonkolleite nanocrystal slurry by which gritty feel and white residues on the skin were dissolved. The results of Table 5 revealed that: polyethylene glycol is also suitably used as the dispersant; and, particularly, polyethylene glycol 6000 (PEG6000) is suitable for improving dispersibility and dissolving gritty feel and white residues on the skin. On the other hand, in the case of using polyethylene glycol 1000, propylene glycol 1-methoxy-2-acetate, or ethoxyethyl acetate as the dispersant, dispersibility was not improved, and furthermore, the simonkolleite crystals were insufficiently downsized and were difficult to finely pulverize into a nano-level size.

The results of Tables 4 and 5 revealed that the simonkolleite crystals are downsized to nanoparticles having a 90% cumulative particle size (D₉₀) of 800 nm or smaller, whereby gritty feel and white residues on the skin are reduced, and the resulting slurry is excellent in feeling of use. These results further indicated that the simonkolleite crystals are downsized to nanoparticles having a 90% cumulative particle size (D₉₀) of 550 nm or smaller and a 50% cumulative particle size (D₅₀) of 150 nm or smaller, whereby gritty feel and white residues on the skin are dissolved, and the resulting slurry offers much better feeling of use as a cosmetic product.

In the case of using a high-pressure homogenizer as a pulverizer for a downsizing treatment condition, difference in type of the chamber had little influence, and the downsizing treatment was performed at a similar level both for the ball collision-type chamber and for the slit-type chamber. Increase in the number of passes was confirmed to attain downsizing into a smaller size. As seen, not only a high-pressure homogenizer as a pulverizer but a bead mill as a pulverizer can be suitably used (Test 5-8) .

### [Example 6]

### 7. Study on dispersibility of simonkolleite nanocrystal slurry - (2)

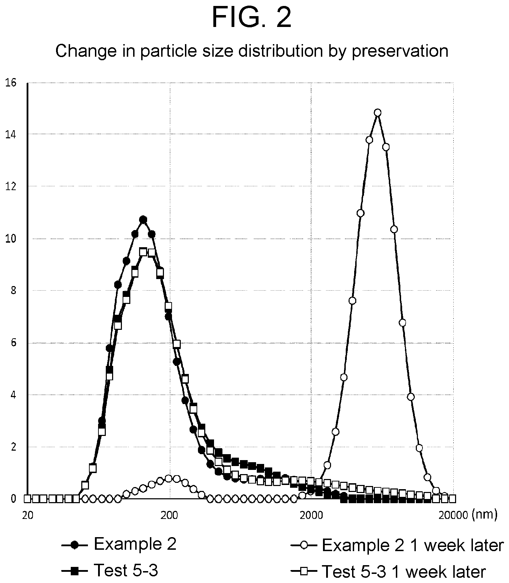

The particle size distribution of simonkolleite crystals was measured as to a sample blended with a dispersant and a sample blended without a dispersant immediately after sample preparation and after storage of the samples at room temperature for 1 week. The sample blended with a dispersant used was the ethanol slurry obtained in Test 5-3 (dispersant: hydroxypropyl cellulose) of Example 5. The sample blended without a dispersant used was the aqueous slurry obtained in Example 2. The results are shown in Figure 2.

As is evident from Figure 2, simonkolleite particles in the sample of Example 2 blended without a dispersant aggregated into 10 µm particles among which 200 nm or smaller particles were hardly observed. On the other hand, the slurry sample of Test 5-3 of Example 5 blended with hydroxypropyl cellulose as the dispersant exhibited little change in particle size distribution of the simonkolleite nanoparticles, and maintained the same particle size distribution as that immediately after sample preparation. This revealed that the dispersant is markedly effective not only for improvement in dispersibility of the slurry but for maintenance of the nano level size of the simonkolleite particles and stability on storage.

### [Example 7]

### 8. Study on method for producing aqueous slurry of simonkolleite nanocrystals - (1)

In the present invention, an event was found to occur easily in which in the case of selecting water as a dispersion medium for simonkolleite crystals, the downsizing treatment of simonkolleite particles is difficult, for example, as shown in Test 7-1 below. Accordingly, various studies were made on a method for producing an aqueous slurry of simonkolleite nanocrystals using water selected as a dispersion medium.

### (Test 7-1)

To 840 g of ion-exchange water, 160 g of simonkolleite (JFE Mineral & Alloy Co., Ltd., lot No: TOL81) was added and mixed, and the mixture was stirred at a stirring rate of 300 to 400 rpm for 3 minutes using a propeller stirrer. The aqueous slurry (aqueous suspension) of simonkolleite crystals thus stirred was introduced to a high-pressure homogenizer as a wet pulverizer (Star Burst Labo, a product of Sugino Machine Ltd.). Although downsizing treatment was attempted under conditions of chamber used: ball collision chamber, chamber nozzle diameter: 0.14 mm, and pressure: 245 MPa, the downsizing treatment was impossible due to the clogging in a pipeline arise from such as adhesion. The results are shown in Table 6.

### (Test 7-2)

In 121.2 g of ion-exchange water, 4.8 g of hydroxypropyl cellulose was dissolved as a dispersant. To this solution, 24 g of simonkolleite (JFE Mineral & Alloy Co., Ltd., lot No: TOL81) was added to obtain a suspension. This suspension was stirred at a stirring rate of 500 to 1000 rpm for 2 hours using a propeller stirrer. The aqueous slurry (aqueous suspension) of simonkolleite crystals thus stirred was introduced to a high-pressure homogenizer as a wet pulverizer (Star Burst Mini, a product of Sugino Machine Ltd.) and subjected to downsizing treatment under conditions of chamber used: ball collision chamber, chamber nozzle diameter: 0.1 mm, pressure: 245 MPa, and the number of passes: 30 passes to obtain an aqueous slurry. The particle size measurement of simonkolleite crystals and various sample evaluations were performed as to this slurry sample. The results are shown in Table 6.

### (Test 7-3)

To 121.2 g of ion-exchange water, 24 g of simonkolleite (JFE Mineral & Alloy Co., Ltd., lot No: TOL81) was added to obtain a suspension. This suspension was stirred for 4 hours using a magnetic stirrer, then left standing overnight, and warmed up to water. To this suspension, 4.8 g of polyethylene glycol 6000 (PEG-6000P, a product of Sanyo Chemical Industries, Ltd.) was added as a dispersant, and the mixture was stirred at a stirring rate of 500 to 1000 rpm for 3 hours using a propeller stirrer. The aqueous slurry (aqueous suspension) of simonkolleite crystals thus stirred was subjected to downsizing treatment using the same conditions and method as in Test 7-2 to obtain an aqueous slurry. The particle size measurement of simonkolleite crystals and various sample evaluations were performed as to this slurry sample. The results are shown in Table 6.

### (Test 7-4)

In 168 g of ion-exchange water, 2.56 g of polyethylene glycol 6000 (PEG-6000P, a product of Sanyo Chemical Industries, Ltd.) was dissolved as a dispersant. To this solution, 32 g of simonkolleite (JFE Mineral & Alloy Co., Ltd., lot No: TOL81) was added to obtain a suspension. This suspension was introduced to a high-pressure homogenizer as a wet pulverizer (Star Burst Mini, a product of Sugino Machine Ltd.). Although downsizing treatment was attempted under conditions of chamber used: ball collision chamber, chamber nozzle diameter: 0.1 mm, and pressure: 245 MPa, the downsizing treatment was impossible due to the clogging in a pipeline arise from, such as adhesion. The results are shown in Table 6.

### (Test 7-5)

In 168 g of ion-exchange water, 2.56 g of polyethylene glycol 400 was dissolved as a dispersant. To this solution, 32 g of simonkolleite (JFE Mineral & Alloy Co., Ltd., lot No: TOL81) was added to obtain a suspension. This suspension was introduced to a high-pressure homogenizer as a wet pulverizer (Star Burst Mini, a product of Sugino Machine Ltd.). Although downsizing treatment was attempted under conditions of chamber used: ball collision chamber, chamber nozzle diameter: 0.1 mm, and pressure: 245 MPa, the downsizing treatment was impossible due to the clogging in a pipeline arise from adhesion, of a pipeline. The results are shown in Table 6.

### (Test 7-6)

In 760 g of ion-exchange water, 40 g of hydroxypropyl cellulose was dissolved as a dispersant. To this solution, 200 g of simonkolleite (JFE Mineral & Alloy Co., Ltd., lot No: S1J201) was added to obtain a suspension. This suspension was stirred at a stirring rate of 500 to 1000 rpm for 5.5 hours using a propeller stirrer. This aqueous slurry (aqueous suspension) of simonkolleite crystals was introduced to a high-pressure homogenizer as a wet pulverizer (Star Burst Labo, a product of Sugino Machine Ltd.) and subjected to downsizing treatment under conditions of chamber used: slit-type chamber, chamber nozzle diameter: 0.16 mm, pressure: 150 MPa, and the number of passes: 50 passes to obtain an aqueous slurry. The particle size measurement of simonkolleite crystals and various sample evaluations were performed as to this slurry sample. The results are shown in Table 7.

### (Test 7-7)

In 1760 g of ion-exchange water, 40 g of hydroxypropyl cellulose was dissolved as a dispersant. To this solution, 200 g of simonkolleite (JFE Mineral & Alloy Co., Ltd., lot No: S1J201) was added to obtain a suspension. This suspension was stirred at a stirring rate of 500 to 1000 rpm for 3 hours using a propeller stirrer and then left standing overnight. This aqueous slurry (aqueous suspension) of simonkolleite crystals was introduced to a high-pressure homogenizer as a wet pulverizer (Star Burst Labo, a product of Sugino Machine Ltd.) and subjected to preliminary milling treatment under conditions of chamber used: slit-type chamber, chamber nozzle diameter: 0.16 mm, pressure: 150 MPa, and the number of passes: 5 passes. After the preliminary milling treatment, the chamber of the wet pulverizer was changed to another slit-type chamber (model number: ESC-101, its nozzle diameter was presumably smaller than that of the chamber used in the preliminary milling treatment), and downsizing treatment was performed under conditions of pressure: 150 MPa and the number of passes: 50 passes to obtain an aqueous slurry. The particle size measurement of simonkolleite crystals and various sample evaluations were performed as to this slurry sample. The results are shown in Table 7.

### (Test 7-8)

In 765.6 g of ion-exchange water, 17.4 g of polyethylene glycol 6000 (PEG-6000P, a product of Sanyo Chemical Industries, Ltd.) was dissolved as a dispersant. To this solution, 87 g of simonkolleite (JFE Mineral & Alloy Co., Ltd., lot No: S1J201) was added to obtain a suspension. This suspension was stirred at a stirring rate of 500 to 1000 rpm for 2 hours using a propeller stirrer. The aqueous slurry (aqueous suspension) of simonkolleite crystals thus stirred was subjected to preliminary milling treatment and downsizing treatment using the same conditions and method as in Test 7-7 to obtain an aqueous slurry. The particle size measurement of simonkolleite crystals and various sample evaluations were performed as to this slurry sample. The results are shown in Table 7.

### (Test 7-9)

In 880 g of ion-exchange water, 10 g of hydroxypropyl cellulose and 10 g of polyethylene glycol 6000 (PEG-6000P, a product of Sanyo Chemical Industries, Ltd.) were dissolved as a dispersant. To this solution, 100 g of simonkolleite (JFE Mineral & Alloy Co., Ltd., lot No: S1J201) was added to obtain a suspension. This suspension was stirred at a stirring rate of 500 to 1000 rpm for 2 hours using a propeller stirrer. The aqueous slurry (aqueous suspension) of simonkolleite crystals thus stirred was subjected to preliminary milling treatment and downsizing treatment using the same conditions and method as in Test 7-7 mentioned above except that the number of passes in the downsizing treatment was set to 40 passes to obtain an aqueous slurry. The particle size measurement of simonkolleite crystals and various sample evaluations were performed as to this slurry sample. The results are shown in Table 7.

### (Test 7-10)

In 1043 g of ion-exchange water, 56 g of hydroxypropyl cellulose as a dispersant and 21 g of phenoxyethanol as a preservative were dissolved. To this solution, 280 g of simonkolleite (JFE Mineral & Alloy Co., Ltd., lot No: S1J201) was added to obtain a suspension. This suspension was stirred at a stirring rate of 500 to 1000 rpm for 3 hours using a propeller stirrer. The aqueous slurry (aqueous suspension) of simonkolleite crystals thus stirred was subjected to preliminary milling treatment and downsizing treatment using the same conditions and method as in Test 7-9 to obtain an aqueous slurry. The particle size measurement of simonkolleite crystals and various sample evaluations were performed as to this slurry sample. The results are shown in Table 7.

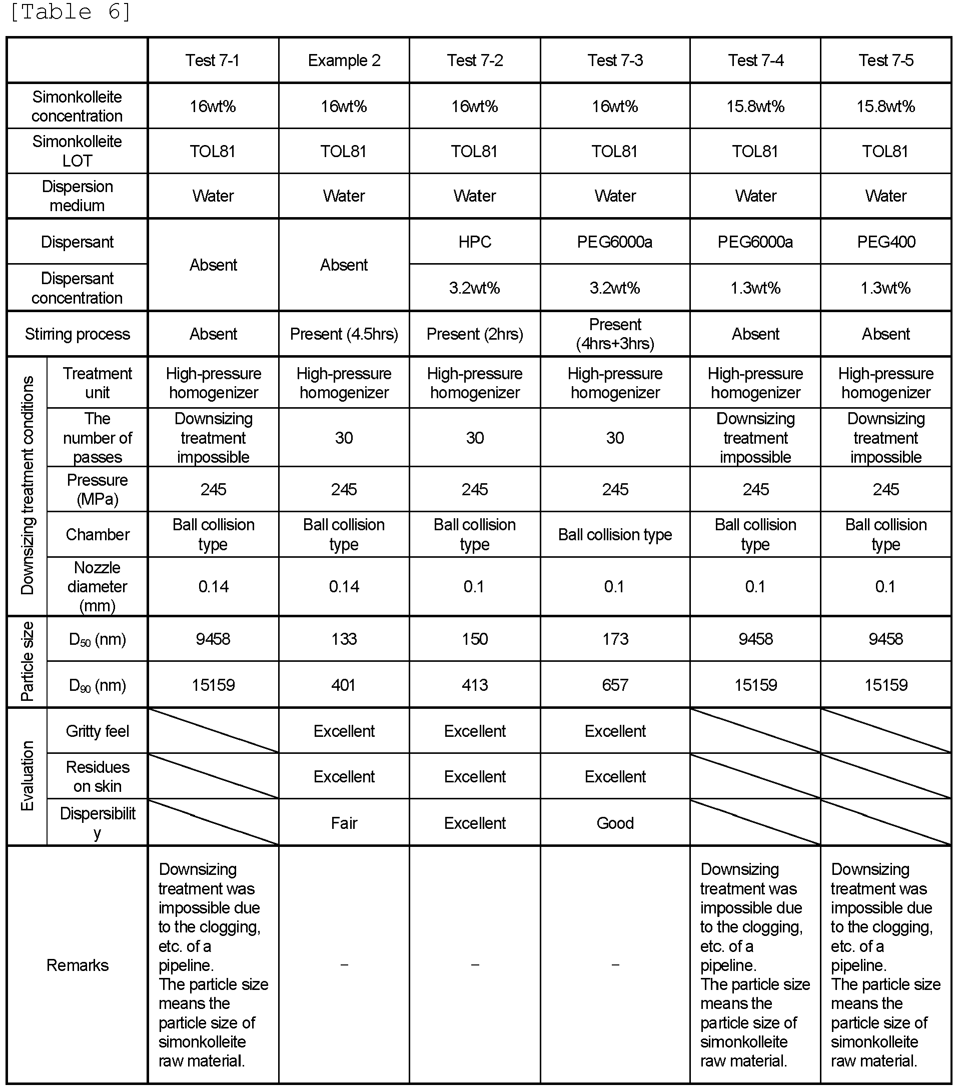

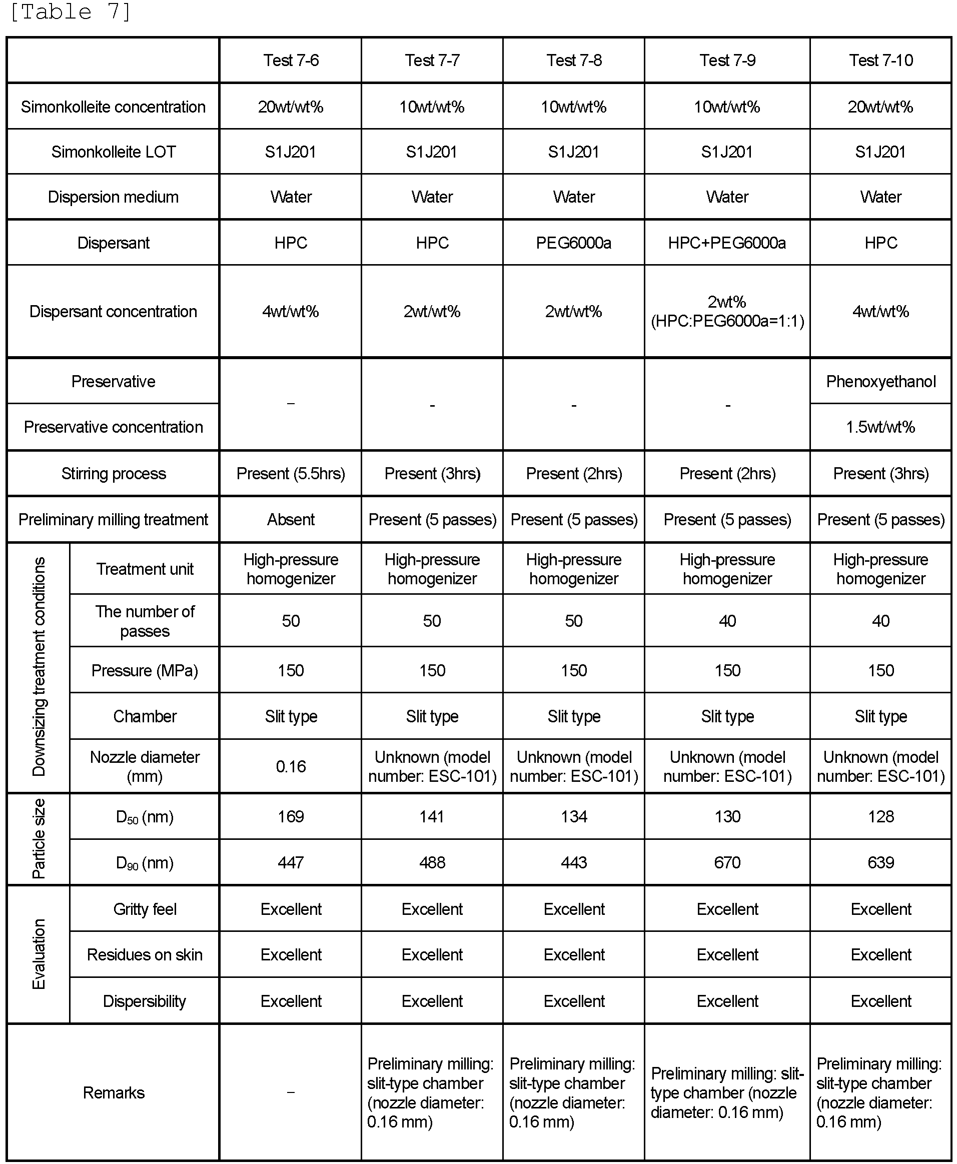

Table 6 also shows the results of Example 2 again. As shown in Table 6, Test 7-1, Test 7-4, and 7-5 without the stirring process caused adhesion presumably caused by the simonkolleite crystals in the high-pressure homogenizer, making the downsizing treatment impossible. On the other hand, as shown in Tables 6 and 7, it became evident that by establishing the stirring process of stirring the suspension containing the simonkolleite crystals and water for a predetermined time before the downsizing treatment process, the simonkolleite crystals were able to be smoothly downsized into a nano-level size even when water was selected as the dispersion medium (Example 2, Test 7-2, Test 7-3, and Tests 7-6 to 7-10). Even in the case of blending a dispersant, the pulverizer operated without any problem through the stirring process, and the simonkolleite crystals were downsized into a nano-level size (Test 7-2, Test 7-3, and Tests 7-6 to 7-10). Even in the case of selecting water as the dispersion medium, the high-pressure homogenizer as a pulverizer was confirmed to downsize the crystals into a favorable level using a chamber of any type (ball collision-type chamber and slit-type chamber). It further became evident that by performing the downsizing treatment after rough milling treatment with a small number of passes in advance as the preliminary milling treatment, the resulting slurry stably had a smaller 50% cumulative particle size (D₅₀) (150 nm or smaller) and was evaluated as being excellent in terms of gritty feel and residues on the skin and dispersibility (Tests 7-7 to 7-10). Even in the case of blending the preservative phenoxyethanol into the slurry, the sample was still excellent in various evaluations without influence on the level of downsizing the simonkolleite crystals (Test 7-10).

### [Example 8]

### 9. Study on method for producing aqueous slurry of simonkolleite nanocrystals - (2)

A study was made on whether to be able to produce an aqueous slurry of simonkolleite nanocrystals by converting the dispersion medium of an ethanol slurry of simonkolleite nanocrystals to water. First, 32 g of ion-exchange water was added to 20 g of the ethanol slurry obtained in Test 5-3 of Example 5. Ethanol was evaporated therefrom by steam distillation under reduced pressure in a rotary evaporator to obtain 27.11 g of an aqueous slurry. Ion-exchange water was added thereto to adjust the amount to 32 g to obtain an aqueous slurry containing 10 w/w% of simonkolleite nanocrystals. 25 g of this product was diluted into 50 mL with ion-exchange water to obtain an aqueous slurry containing 5 w/v% of simonkolleite nanocrystals and 1 w/v% of hydroxypropyl cellulose (Table 8).

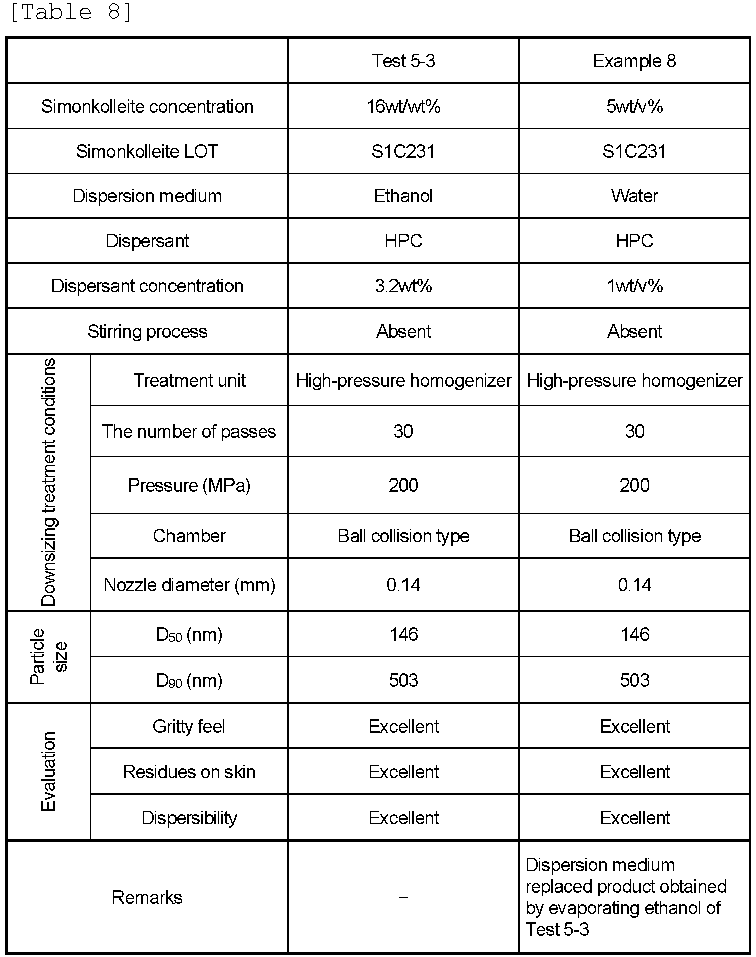

As shown in Table 8, the dispersion medium was able to be converted from ethanol to water by evaporating the ethanol by steam distillation from the ethanol slurry of simonkolleite nanocrystals. In the case of selecting ethanol as the dispersion medium, no stirring process is necessary. Therefore, a production method can be proposed which involves preparing a simonkolleite nanocrystal slurry as an ethanol slurry, and evaporating ethanol, if necessary, to prepare an aqueous slurry.

### [Example 9]

### 10. Study on hair growth effect of simonkolleite crystals - (2)

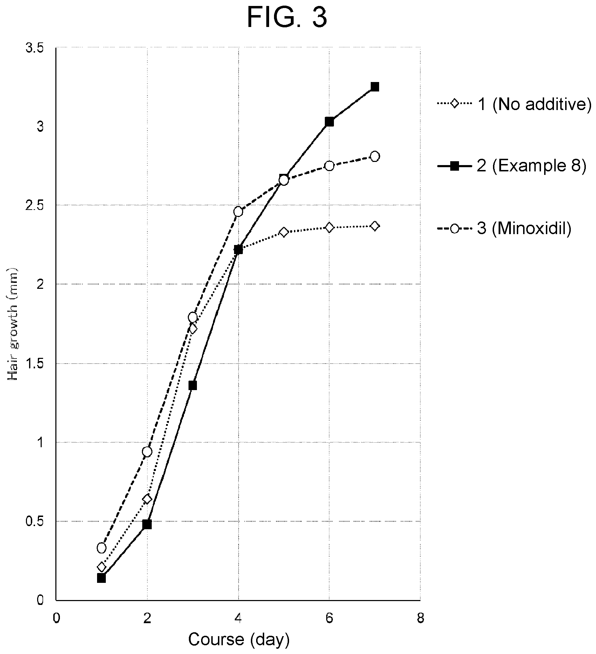

The organ culture of rat whisker follicle was performed using the sample blended with a dispersant prepared in Example 8 to examine a hair growth promoting effect of simonkolleite. The test was conducted by the same method as in Example 4 mentioned above. Table 9 below shows a culture solution used in each test group (Tests 1 to 3). The culture was performed for 7 days, and hair elongation was observed and recorded as change in whole length from the starting point of melanocyte to the hair cut portion and length of the exposed hair portion from skin surface to the hair cut portion under a stereomicroscope. The results are shown in Figure 3.

**[Table 9]**

| Test No. | Sample added to culture solution | Sample concentration in culture solution |
|---|---|---|
| 1 | Negative control: no additive | - |
| 2 | Example 8: aqueous suspension of simonkolleite nanocrystals + dispersant | Simonkolleite concentration: 5 µg/mL (0.01% of Example 8) |
| 3 | Positive control: minoxidil | Minoxidil concentration: 50 µM |

As in the results of Figure 1 about Example 4, as shown in Figure 3, only the test group (Test 2) supplemented with the sample containing the simonkolleite nanocrystals and the dispersant in Example 8 exhibited marked hair elongation since 5 days after the start of the test. This elongation effect stood out as compared not only with the negative control test group (Test 1) but with the positive control minoxidil test group (Test 3). These results indicate that the simonkolleite nanocrystals also have a hair elongation effect, as in Example 4.

### [Example 10]

### 11. Preparation of cosmetic for hair growth

12.5% by weight of the simonkolleite nanocrystal slurry obtained in Test 5-8 of Example 5 was mixed with 5% by weight of ethanol, 10% by weight of dipropylene glycol, 2% by weight of propylene glycol-10 methyl glucose, 20% by weight of butylene glycol, 1% by weight of bis-glyceryl ascorbate, 1% by weight of 1,2-hexanediol, 1% by weight of glycine, 1% by weight of arginine, 0.5% by weight of glycerin, 0.5% by weight of hydroxyethyl cellulose, 0.5% by weight of dextran, 0.5% by weight of phenoxyethanol, 0.2% by weight of *Citrus junos* fruit extract, 0.2% by weight of *Rehmannia chinensis* root extract, 0.2% by weight of acetyl tetrapeptide-3, 0.2% by weight of *Trifolium pratense* flower extract, 0.2% by weight of biotin, 0.2% by weight of riboflavin, 0.2% by weight of pyridoxin HCl, 0.2% by weight of dipotassium glycyrrhizate, 0.2% by weight of sodium acetyl hyaluronate, 0.2% by weight of hydrolyzed elastin, 0.2% by weight of cholesterol, 0.2% by weight of PEG-40 hydrogenated castor oil, 0.2% by weight of polyglyceryl-10 myristate, 0.2% by weight of diphenyl dimethicone, 0.05% by weight of bergamot fruit oil, 0.05% by weight of *Boswellia carterii* oil, 0.05% by weight of lime juice, 0.05% by weight of orange juice, 0.05% by weight of lemon juice, 0.05% by weight of *Crataegus cuneata* fruit extract, 0.05% by weight of *Zizyphus jujuba* fruit extract, 0.05% by weight of grapefruit extract, 0.05% by weight of apple extract, and a balance of water to obtain a lotion for hair growth in the form of a slightly viscous light pink-white suspension with fresh aroma (component content of the simonkolleite nanocrystals in the lotion: 2% by weight of, component content of polyethylene glycol 6000: 0.2% by weight of).

The present invention is not limited by the embodiments or the examples described above, and also includes various forms with changed or modified designs in its technical scope, without departing from the spirit of the invention described in the scope of claims.

### Industrial Applicability

The cosmetic for hair growth, the cosmetic raw material, and the cosmetic of the present invention have an excellent hair growth effect and are also excellent in feeling of use, ease of handling, and stability, and as such, are widely utilized in the fields of beauty care and hair care.

## Claims

1. A cosmetic for hair growth comprising simonkolleite crystals as an active ingredient.

2. The cosmetic for hair growth according to claim 1, wherein an amount of the simonkolleite crystals blended is 0.001% by weight to 10% by weight.

3. The cosmetic for hair growth according to claim 1 or 2, wherein the simonkolleite crystals are nanoparticles having a 90% cumulative particle size (D₉₀) of 800 nm or smaller in a volume-based particle size distribution obtained by a laser diffraction scattering method.

4. The cosmetic for hair growth according to any one of claims 1 to 3, further comprising a dispersion medium and a dispersant, wherein
the dispersant is at least one compound selected from the group consisting of polyethylene glycol, polypropylene glycol, an ethylene oxide-propylene oxide copolymer, hydroxypropyl cellulose, hydroxypropyl methylcellulose, and hydroxyethyl cellulose.

5. A cosmetic raw material or a cosmetic comprising nanoparticles of simonkolleite crystals.

6. The cosmetic raw material or the cosmetic according to claim 5, wherein the nanoparticles of simonkolleite crystals have a 90% cumulative particle size (D₉₀) of 800 nm or smaller in a volume-based particle size distribution obtained by a laser diffraction scattering method.

7. The cosmetic raw material or the cosmetic according to claim 5 or 6, wherein the nanoparticles of simonkolleite crystals have a 50% cumulative particle size (D₅₀) of 250 nm or smaller in a volume-based particle size distribution obtained by a laser diffraction scattering method.

8. The cosmetic raw material or the cosmetic according to any one of claims 5 to 7, further comprising water, a water-miscible organic solvent, or a combination thereof as a dispersion medium.

9. The cosmetic raw material or the cosmetic according to claim 8, further comprising a dispersant.

10. The cosmetic raw material or the cosmetic according to claim 9, wherein the dispersant is at least one compound selected from the group consisting of polyethylene glycol, polypropylene glycol, an ethylene oxide-propylene oxide copolymer, hydroxypropyl cellulose, hydroxypropyl methylcellulose, and hydroxyethyl cellulose.

11. The cosmetic raw material or the cosmetic according to any one of claims 5 to 10, wherein an amount of the simonkolleite crystals blended is 0.001% by weight to 30% by weight.

12. The cosmetic raw material or the cosmetic according to any one of claims 5 to 11, wherein the cosmetic raw material or the cosmetic is for hair growth or for scalp care.

13. A method for producing a cosmetic raw material or a cosmetic, comprising:
a mixing process of mixing simonkolleite crystals with a dispersion medium to obtain a suspension;
a stirring process of stirring the suspension for 1 hour or longer; and
a downsizing treatment process of introducing the suspension after the stirring process to a pulverizer, and downsizing the simonkolleite crystals contained in the suspension into a nano-level size.

14. The method for producing a cosmetic raw material or a cosmetic according to claim 13, wherein in the mixing process or the stirring process, the suspension is further mixed with a dispersant.

15. A method for producing a cosmetic raw material or a cosmetic, comprising:
a mixing process of mixing simonkolleite crystals with a dispersion medium having a water-miscible organic solvent to obtain a suspension;
a downsizing treatment process of introducing the suspension to a pulverizer, and downsizing the simonkolleite crystals contained in the suspension into a nano-level size; and
a process of evaporating the water-miscible organic solvent contained in the suspension after the downsizing treatment.

16. The method for producing a cosmetic raw material or a cosmetic according to claim 15, wherein in the mixing process, the suspension is obtained by further mixing with a dispersant.
